(19) Europäisches Patentamt

European Patent Office

Office européen des brevets

(11) **EP 3 331 617 B1**

(12) **FASCICULE DE BREVET EUROPEEN**

(45) Date de publication et mention de la délivrance du brevet:
**25.08.2021 Bulletin 2021/34**

(21) Numéro de dépôt: **16760528.6**

(22) Date de dépôt: **29.07.2016**

(51) Int Cl.:
***A61Q 19/10*** (2006.01)   ***A61K 8/9789*** (2017.01)

(86) Numéro de dépôt international:
**PCT/FR2016/051992**

(87) Numéro de publication internationale:
**WO 2017/021641 (09.02.2017 Gazette 2017/06)**

(54) **COMPOSITION COSMETIQUE EXFOLIANTE COMPRENANT DES MORCEAUX D'AGRUMES CONFITS**

ABSCHÄLENDE KOSMETISCHE ZUSAMMENSETZUNG MIT STÜCKEN VON KANDIERTEN ZITRUSFRÜCHTEN

EXFOLIATING COSMETIC COMPOSITION INCLUDING PIECES OF CANDIED CITRUS FRUIT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **03.08.2015 FR 1557473**

(43) Date de publication de la demande:
**13.06.2018 Bulletin 2018/24**

(73) Titulaire: **LvmH Recherche**
**45800 Saint-Jean De Braye (FR)**

(72) Inventeurs:
• **KURFURST, Chantal**
**45800 Saint Jean de Braye (FR)**

• **GERARD, Véronique**
**45000 Orléans (FR)**
• **SCATTARELLI, Dominique**
**45140 Saint Jean de la Ruelle (FR)**

(74) Mandataire: **Cabinet Beau de Loménie**
**158, rue de l'Université**
**75340 Paris Cedex 07 (FR)**

(56) Documents cités:
**WO-A1-2008/078332     FR-A- 1 463 569**
**FR-A1- 2 769 178     US-A- 2 611 708**
**US-A1- 2004 091 446**

**Description**

L'invention concerne un nouvel agent cosmétique exfoliant, des compositions cosmétiques le contenant, et un procédé de soin de la peau qui le met en œuvre.

L'invention concerne plus particulièrement l'utilisation de morceaux de fruits ayant subi un procédé de confisage comme agent exfoliant de la peau.

ETAT DE LA TECHNIQUE

On connaît divers moyens d'exfolier les cellules mortes de la peau.

On peut tout d'abord avoir recours à des moyens mécaniques qui utilisent des particules solides de faible granulométrie, comme par exemple des poudre de pierre ponce, de polyéthylène ou de nylon, des fibres de polyamide, des particules de quartz ou de riz, des broyats de coques ou de noyaux de fruits tels que par exemple des morceaux de coquilles d'amandes ou de noix, de noyaux de pêches ou d'abricots. D'autres moyens mécaniques existent comme par exemple un gant ou un matériau constitué de fibres de crin.

On peut également agir par voie chimique à l'aide d'agents cosmétiques qui provoquent l'élimination des cellules mortes de la couche cornée en réduisant chimiquement leur cohésion, facilitant ainsi leur détachement (action kératolytique). A titre d'exemple, on peut citer des acides alpha-hydroxylés (acides de fruits) tels que l'acide glycolique, l'acide lactique, l'acide malique, l'acide tartrique ou les acides béta-hydroxylés tels que l'acide salicylique.

On peut également combiner des moyens mécaniques et chimiques dans des compositions cosmétiques exfoliantes.

On obtient l'effet d'exfoliation recherché en appliquant les compositions qui comprennent les agents exfoliants susmentionnés par des massages plus ou moins vigoureux. L'exfoliation des cellules mortes de la peau doit cependant être réalisée de façon contrôlée. En effet, l'ensemble de ces moyens, éventuellement combinés, peut provoquer une abrasion trop forte de la couche cornée, se traduisant par l'apparition de rougeurs ou de zones hypersensibles. Une exfoliation trop agressive peut alors induire la perte même partielle de la fonction naturelle de barrière et de protection de la couche superficielle de l'épiderme vis-à-vis d'agents externes agressifs, microbiologiques ou environnementaux, pouvant provoquer l'apparition d'infections cutanées ou des sensations désagréables.

Il existe donc un besoin pour de nouveaux agents exfoliants d'origine naturelle produisant un effet exfoliant agréable à l'application, et ne risquant pas de produire d'abrasion de la peau trop importante, même en cas de massage intense.

Il a été découvert à présent qu'il est possible d'obtenir un effet exfoliant au niveau cutané en utilisant un matériau d'origine fruitière ayant une texture souple, contrairement aux morceaux de coques ou de noyaux de fruits couramment utilisés. Ce nouvel agent exfoliant naturel produit un effet mécanique efficace, tout en restant particulièrement agréable à l'application, et sans risquer de générer des irritations.

Les inventeurs ont trouvé de manière totalement inattendue, qu'il est possible d'obtenir une exfoliation douce, respectueuse de la couche superficielle de l'épiderme, en utilisant des morceaux de fruits ayant été traités par un procédé de confisage.

Le confisage de morceaux de fruits, et plus particulièrement de morceaux comprenant au moins en partie de l'enveloppe du fruit, permet de transformer la texture ferme d'un fruit frais en une texture souple qui permet une exfoliation douce des cellules mortes de la couche cornée par massage de la peau, tout en procurant un effet agréable.

Il est particulièrement intéressant de disposer ainsi d'un agent exfoliant mécanique qui ne procure aucun effet agressif au niveau cutané notamment une abrasion trop importante.

Le confisage est un procédé de conservation des fruits basé sur le principe de l'osmose, qui vise à remplacer l'eau contenu dans le fruit frais par du sucre. Le fruit est plongé dans un sirop riche en sucre pour provoquer un échange entre l'eau du fruit et le sucre du sirop. Le fruit confit est ainsi simultanément enrichi en sucre et déshydraté. Le confisage est un procédé très largement utilisé dans l'industrie alimentaire pour tout type de fruits qui peuvent ensuite être consommés directement ou bien être utilisés dans des préparations culinaires.

L'utilisation de fruits dans des compositions cosmétiques se heurte à plusieurs difficultés.

En premier lieu, une forte teneur en sucre dans un milieu aqueux crée des conditions propices au développement bactérien, ce qui nuit à la stabilité et à la conservation de produits cosmétiques aqueux.

En second lieu, certains fruits contiennent des composés néfastes pour la peau qui empêchent leur utilisation dans des compositions cosmétiques. Dans le cas des agrumes, l'écorce comprend des composés de type furocoumarine qui sont des agents photosensibles toxiques. On trouve notamment dans les zestes d'agrumes le bergaptène qui est photosensibilisant. Un contact prolongé avec ces composés ou un végétal en contenant, suivi d'une exposition au soleil, peut provoquer des dermatites aiguës. Or il a été constaté de façon inattendue par les inventeurs que le procédé de confisage appliqué à des écorces d'agrumes permet d'éliminer les furocoumarines présentes dans l'écorce.

L'utilisation en cosmétique de morceaux d'agrumes ou d'extraits de morceaux d'agrumes, plus particulièrement de morceaux comprenant au moins en partie de l'écorce dudit agrume, est donc rendue possible par la mise en œuvre d'un procédé de confisage. Le confisage élimine simultanément l'eau et les furocoumarines incriminées.

Le procédé de confisage rend donc des écorces d'agrumes utilisables dans des compositions cosmétiques sans risque.

Il permet également d'améliorer la stabilité et la conservation des produits cosmétiques comprenant de telles écorces d'agrumes.

Le confisage permet en outre de modifier la texture des morceaux de fruits. Le confisage des écorces d'agrumes permet en particulier d'obtenir une texture plus molle et rend les morceaux confits d'écorce d'agrumes utilisables comme agent exfoliant mécanique.

L'effet exfoliant obtenu par ces morceaux confits d'agrumes permet une exfoliation douce des cellules mortes de la couche cornée, particulièrement intéressante pour les peaux fragiles ou fragilisées telles que les peaux matures ou des peaux présentant des signes de sécheresse cutanée ou les peaux atopiques.

US 2004/0091446 A1 décrit des compositions démaquillantes à base de tensioactifs synthétiques, d'émollients hydrophiles et de particules exfoliantes, lesdites compositions comprenant au moins 80% de particules, dont la longueur de l'axe principal mesure entre 100 et 1000 $\mu$m. Ce document mentionne le zeste de Citrus Tangeria (Tangerine) parmi plus d'une centaine d'exfoliants, sans que cet agrume ne soit traité par déshydratation osmotique ménagée dans un sirop de sucre.

BUTS DE L'INVENTION

La présente invention a ainsi pour but principal de fournir un nouvel agent cosmétique exfoliant sous la forme de morceaux de fruits confits.

La présente invention a plus particulièrement pour objet de fournir un nouvel agent exfoliant sous la forme de morceaux d'agrume confits comprenant au moins en partie l'écorce et/ou l'épicarpe dudit agrume.

L'invention a aussi pour objet un soin cosmétique comprenant un agent exfoliant sous forme d'un morceau de fruit confit, en particulier d'agrume.

L'invention a aussi pour but de fournir une méthode de soin cosmétique visant à une exfoliation douce des cellules mortes de la peau par un massage à l'aide de morceaux de fruits confits ou de compositions cosmétiques comprenant lesdits morceaux confits.

DESCRIPTION DETAILLEE DE L'INVENTION

L'invention concerne l'utilisation en cosmétique de morceaux de fruits confits en tant qu'agent exfoliant mécanique de la peau.

Les morceaux de fruits confits peuvent être utilisés seuls ou dans une composition cosmétique comprenant au moins un excipient cosmétiquement acceptable, ou encore dans un kit comprenant une composition cosmétique.

Selon une première variante, on utilise les morceaux de fruits confits seuls pour masser la peau à l'aide desdits morceaux de fruits.

Dans une deuxième variante, les morceaux de fruits confits sont imprégnés d'une composition cosmétique comprenant au moins un excipient cosmétiquement acceptable.

Selon une troisième variante, les morceaux de fruits confits sont ajoutés comme agent exfoliant mécanique dans une composition cosmétique comprenant au moins un excipient cosmétiquement acceptable.

Dans une quatrième variante, les morceaux de fruits confits sont présentés dans un kit dans lequel ils sont conditionnés séparément d'une composition cosmétique comprise dans le kit. Dans le cas où les morceaux de fruit et la composition sont conditionnés séparément, on peut déposer manuellement la composition sur la peau et l'étaler à l'aide d'au moins un morceau de fruit tout en massant.

Dans les deuxième et quatrième variantes, les morceaux de fruits confits sont utilisés pour masser la peau et produire ainsi son exfoliation. Ils servent également de supports applicateurs de la composition cosmétique.

Dans le cas où les morceaux de fruit sont imprégnés de composition, on peut les appliquer directement sur la peau et masser la peau.

Un premier objet de l'invention concerne l'utilisation de morceaux d'agrume obtenus par déshydratation osmotique ménagée dudit agrume dans un sirop de sucre, pour l'exfoliation mécanique des cellules mortes de l'épiderme de la peau d'une personne, lesdits morceaux d'agrume comprenant l'écorce et/ou l'épicarpe dudit agrume.

Un deuxième objet de l'invention est un soin cosmétique exfoliant comprenant des morceaux d'agrume obtenus par déshydratation osmotique ménagée dudit agrume dans un sirop de sucre, à titre d'agent d'exfoliation mécanique, lesdits morceaux confits comprenant l'écorce et/ou l'épicarpe dudit agrume.

La composition du soin cosmétique de l'invention contient un liquide choisi dans le groupe constitué de l'eau, une huile et un mélange d'eau et d'huile, et au moins un copolymère acrylique en tant que gélifiant hydrophile.

Selon un troisième objet, l'invention concerne un kit cosmétique exfoliant comprenant

a) une composition cosmétique comprenant un liquide choisi dans le groupe constitué de l'eau, une huile et un mélange d'eau et d'huile, et au moins un excipient choisi parmi les surfactants et les gélifiants, et

b) des morceaux d'agrume ayant de préférence une taille comprise entre 0,1 mm et 5 cm, obtenus par déshydratation osmotique ménagée dudit agrume dans un sirop de sucre,

la composition et les morceaux d'agrume étant conditionnés de façon séparée dans un même emballage.

Un quatrième objet de la présente invention est un procédé d'exfoliation de la peau comprenant les étapes consistant à

- appliquer, sur la peau d'une personne, des morceaux d'agrume ayant de préférence une taille comprise entre 0,1 mm et 5 cm, obtenus par déshydratation osmotique ménagée dudit agrume dans un sirop de sucre,
- masser la peau recouverte desdits morceaux par frottement de la surface de la peau, de manière à exfolier mécaniquement les cellules mortes qui s'y trouvent,
- rincer ou essuyer la peau pour ôter les morceaux confits d'agrume et les cellules mortes exfoliées.

le fruit utilisé est un agrume. Les agrumes sont les fruits de végétaux appartenant à la famille des *Rutaceae,* en particulier des genres *Citrus, Fortunella, Microcitrus, Eremocitrus* et *Poncirus,* ou bien encore des végétaux hybrides obtenus par croisements de variétés ou d'espèces différentes d'espèces telles que *Citrofortunella.*

Les agrumes peuvent être par exemple choisis parmi les citrons, les clémentines, les kumquats, les bergamotes, les limes, les limettes, les mandarines, les oranges, les pamplemousses, les pomelos, les tangerines, les combavas, les yuzus, les cédrats et le calamondin.

L'agrume utilisé pour le confisage est constitué d'une part de quartiers qui contiennent de la pulpe, et d'autre part d'une écorce, également appelée péricarpe. L'écorce est elle-même constituée de deux couches concentriques. La couche superficielle présente une texture rugueuse et résistante, de couleur vive souvent jaune orangé, riche en flavonoïdes, est nommée épicarpe, ou zeste en cuisine. La couche interne, blanche et spongieuse, est le mésocarpe.

La texture de l'épicarpe du fruit est ramollie par le procédé de confisage mais conserve ses caractéristiques de rugosité et de résistance qui permettent de produire un effet exfoliant sans abrasion excessive de la peau tout en restant agréable à l'application.

Au sens de l'invention, le terme « écorce » utilisé dans la description désigne l'épicarpe de l'agrume ou bien l'épicarpe sur lequel on a laissé le mésocarpe et éventuellement une partie de la pulpe.

Les morceaux confits d'agrumes sont caractérisés en ce qu'ils sont formés au moins en partie de l'écorce de l'agrume.

Dans une variante particulièrement préférée, les morceaux de fruits confits sont constitués d'écorces confites d'agrume.

Pour la mise en oeuvre de l'invention, on peut indifféremment utiliser des morceaux d'une variété unique d'agrumes ou de plusieurs variétés en mélange.

De même, pour une variété d'agrume, on peut indifféremment utiliser une seule fraction confite de l'agrume, par exemple l'écorce, ou plusieurs fractions en mélange.

Dans le cas de mélanges de morceaux de différents fruits et/ou de différentes fractions desdits fruits, on utilise au moins des morceaux confits d'écorce d'au moins un agrume.

Comme expliqué précédemment, les morceaux d'agrumes peuvent être exclusivement formés de l'écorce de l'agrume, mais peuvent aussi comprendre d'autres parties de l'agrume, par exemple un morceau de chair de l'agrume.

Selon une variante préférée, les morceaux d'agrumes sont des morceaux confits d'écorce d'agrume.

Avantageusement, les morceaux d'agrumes ont une taille comprise entre 0,1 millimètre et 5 centimètres.

Ils sont avantageusement obtenus par broyage de lamelles d'écorces de quelques centimètres de longueur découpées dans l'écorce de l'agrume. Ces lamelles peuvent être découpées en morceaux plus petits, avant ou après confisage, en fonction de l'application envisagée.

Le confisage peut ainsi être réalisé sur des morceaux de toute taille, sachant qu'il est possible de réduire la taille des morceaux par un broyage une fois que ceux-ci ont été confits.

Le confisage comprend plusieurs étapes successives connues de l'homme du métier.

La première série d'étapes consiste en la récolte du fruit, son lavage visant à éliminer toute trace de substances étrangères (terre insectes, produits de traitement), éventuellement son blanchiment et sa découpe. La découpe peut être effectuée de manière à obtenir les morceaux de taille voulue juste après l'étape de déshydratation osmotique, ou de manière plus grossière de sorte que les morceaux de fruits confits de taille grossière sont broyés après déshydratation pour obtenir les morceaux de taille voulue.

L'étape de blanchiment est un traitement par lequel on plonge les fruits ou morceaux de fruit dans l'eau portée à ébullition. Ce traitement permet de casser les cloisons cellulaires et facilite la pénétration du sucre dans le fruit. Dans le cas des écorces agrumes, le blanchiment permet également d'en retirer l'amertume, en solubilisant dans l'eau les composés qui en sont responsables. Cette étape peut être répétée plusieurs fois pour retirer toute l'amertume des écorces.

Dans une deuxième série d'étapes, on met les fruits ou morceaux de fruits au contact d'au moins une solution (sirop) de sucre afin d'éliminer l'eau des fruits par osmose.

Le sirop de sucre peut être essentiellement constitué d'eau et d'au moins un sucre. Le sucre est choisi parmi les sucres proprement dits, les dérivés de sucres et les polyols. Le sucre est par exemple choisi parmi le saccharose, le glucose, le sucre inverti (mélange équimolaire de glucose et de fructose obtenu par hydrolyse du saccharose), le dextrose, le sorbitol, le fructose, le miel.

Le sirop peut également comprendre des conservateurs, des colorants et/ou des correcteurs de pH.

Dans le cas où le sirop est très concentré et visqueux, on peut le chauffer légèrement de façon à le fluidifier.

L'étape d'élimination de l'eau des fruits par osmose est de préférence réalisée jusqu'à élimination de toute l'eau du fruit, par exemple en utilisant successivement plusieurs sirops. Le sirop peut être remplacé à intervalles réguliers, typiquement toutes les 24 heures, par un nouveau sirop de concentration en sucre supérieure ou égale à celle du sirop précédent.

Parmi les caractéristiques pouvant influer sur la qualité du fruit après confisage, on peut mentionner la température des bains utilisés pour le confisage, le mode de découpe des fruits, la durée de mise en contact et le nombre de cycles.

Une fois le confisage terminé, on sépare le fruit du sirop, on laisse le sirop excédentaire s'égoutter. Les fruits confits sont refroidis le cas échéant, et éventuellement séchés.

Les morceaux confits peuvent être utilisés tels quels ou bien subir un traitement supplémentaire tel qu'une étape de glaçage afin d'obtenir un aspect brillant, ou bien une étape de broyage afin de réduire à nouveau la taille des morceaux confits.

Les morceaux confits de fruits utilisés dans le cadre de l'invention présentent l'avantage d'être essentiellement dépourvus de composés furocoumarine. Au sens de l'invention, l'expression « essentiellement dépourvus de composés furocoumarine » signifie que les morceaux de fruits confits contiennent moins de 50 ppm de composés furocoumarine, de préférence moins de 20 ppm, de manière plus préférée moins de 10 ppm, de manière plus préférée moins de 5 ppm, de manière plus préférée moins de 4 ppm, de manière préférée moins de 3 ppm, de manière plus préférée moins de 2 ppm. De manière particulièrement avantageuse, les morceaux de fruits confits contiennent moins de 1 ppm de composés furocoumarine.

Les morceaux d'agrumes confits ont par exemple un degré brix compris entre 75 et 85, par exemple de l'ordre de 80. Le degré Brix du sirop de sucre utilisé dans le cadre de la présente invention, correspond à la fraction de sucres présents dans le sirop, c'est-à-dire au pourcentage de matière sèche soluble. Le degré Brix peut être mesuré selon la norme NFV 05109. L'appareil utilisé pour la mesure est un réfractomètre. Suivant la teneur en sucre du jus, la déviation de la lumière du jour par l'échantillon varie et indique par une délimitation colorée le degré Brix. L'unité de mesure est le % (pourcentage de sucre dans le sirop).

Les morceaux d'agrumes confits ont par exemple un pH compris entre 3 et 4. Le pH peut être mesuré selon la norme NFV 1132.

Les morceaux d'agrume confits contiennent du sirop de sucre, dont la teneur peut varier de 20 à 60% en poids. La teneur est par exemple de 50% lorsque le sirop utilisé est un sirop de glucose-fructose.

Comme expliqué précédemment, l'invention vise également un soin cosmétique exfoliant comprenant des morceaux de fruits confits en tant qu'agent exfoliant mécanique. Mais la composition du soin peut comprendre en outre un ou plusieurs autres agents exfoliants mécaniques ou chimiques, comme par exemple de la cire de jojoba.

La composition de l'invention peut notamment comprendre un ou plusieurs agents cosmétiques dont l'effet est complémentaire à celui produit par les morceaux de fruit confit.

Parmi ces agents actifs, on peut citer la D-biotine (Vitamine B7) dermo-protectice et éclaircissante, un extrait d'Ascophyllum Nodosum qui stimule l'enzyme SCCE impliquée dans la desquamation, un extrait d'écorce d'Enantia Chlorantha, qui diminue la sécrétion de sébum et resserre les pores. Parmi les agents cosmétiques, on peut encore citer les acides de fruits (AHA, BHA, AHA greffés) ayant des propriétés exfoliantes, kératolytiques et régénérantes des acides de fruits. Les AHA ont une action d'exfoliation en profondeur liée à la synthèse du collagène du derme. Ils stimulent le renouvellement cellulaire de la peau, réduisent l'épiderme en surface et l'augmentent en profondeur.

On peut encore citer WATER AND ZINC GLUCONATE AND MAGNESIUM ASPARTATE AND COPPER GLUCONATE pour un effet énergisant, VACCINIUM MYRTILLUS FRUIT EXTRACT AND CITRUS MEDICA LIMONUM EXTRACT AND SACCHARUM OFFICINARUM (SUGAR CANE) EXTRACT AND ACER SACCHARUM (SUGAR MAPLE) EXTRACT AND CITRUS AURANTIUM DULCIS (ORANGE) FRUIT EXTRACT qui favorise le renouvellement cellulaire, D PANTHENOL en tant qu'apaisant et hydratant, CITRUS MEDICA LIMONUM FRUIT EXTRACT qui génère une action kératolytique.

Les compositions du soin de l'invention contiennent de l'eau, une huile ou un mélange d'eau et d'huile et peuvent se présenter sous toutes les formes galéniques normalement utilisées pour une application sur la peau d'une personne. Ces compositions sont préparées selon les méthodes usuelles.

Dans le soin cosmétique exfoliant de l'invention, les morceaux d'agrume peuvent être imprégnés d'une composition cosmétique comprenant un liquide choisi dans le groupe constitué de l'eau, d'une huile ou d'un mélange d'eau et d'huile, et au moins un copolymère acrylique en tant que gélifiant hydrophile.

Alternativement, le soin cosmétique exfoliant se présente sous la forme d'une composition cosmétique dans laquelle sont répartis les morceaux d'agrume de manière homogène, ladite composition comprenant un liquide choisi dans le groupe constitué de l'eau, d'une huile ou d'un mélange d'eau et d'huile, et au moins un copolymère acrylique en tant que gélifiant hydrophile.

Le soin exfoliant se présente avantageusement sous la forme d'une composition présentant l'aspect et/ou la texture d'une marmelade. Il s'agit avantageusement d'une composition gélifiée, avantageusement aqueuse dans laquelle on disperse les morceaux confits.

La composition peut être également une émulsion, avantageusement huile-dans-eau ou eau-dans-huile qui présente une texture requise pour une application exfoliante.

Les compositions peuvent contenir des adjuvants habituels dans le domaine cosmétique, tels que les matières grasses, les émulsionnants, les gélifiants hydrophiles ou lipophiles, les actifs hydrophiles ou lipophiles, les conservateurs, les antioxydants, les parfums, les charges, les filtres et les matières colorantes.

Les compositions sont formulées pour une application sur la peau, et présentent un effet particulièrement recherché pour exfolier les cellules mortes en surface de la peau, tout en procurant un effet agréable à l'application.

Les compositions peuvent se présenter par exemple sous la forme de crèmes de soin, de gels de soin, de masques.

Lorsque la composition est une émulsion ou un produit contenant de l'eau et une huile non émulsionnées, la composition comprend une phase grasse comprenant au moins une huile, ainsi que des émulsionnants et coémulsionnants choisis parmi ceux classiquement utilisés dans le domaine cosmétique.

Parmi les huiles utilisables en cosmétique, on peut citer les huiles minérales telles que le polyisobutène hydrogéné et l'huile de vaseline, les huiles végétales issues du beurre de karité, du tournesol ou encore d'amandes d'abricot, les huiles animales, les huiles de synthèse notamment l'huile de Purcellin, le myristate d'isopropyle et le palmitate d'éthyl hexyle, et les huiles fluorées comme par exemple les perfluoropolyéthers. On peut aussi utiliser des alcools gras, des acides gras comme par exemple l'acide stéarique, des cires notamment de paraffine, carnauba ou la cire d'abeilles. On peut aussi utiliser les huiles siliconées et par exemple les cyclométhicone et diméthicone, les cires, résines et gommes siliconées.

Comme émulsionnants, on peut citer par exemple le stéarate de glycérol, le polysorbate 60, le PPG-3 myristyl éther, les émulsionnants siliconés tels que le cétyldiméthicone copolyol et le mono- ou tristéarate de sorbitane, le stéarate de PEG-40, le monostéarate de sorbitane oxyéthyléné (20 OE).

La composition de soin comprend au moins un agent gélifiant hydrophile choisi parmi les copolymères acryliques.

Dans une mise en œuvre préférée, les morceaux confits sont ajoutés dans une composition aqueuse gélifiée.

La viscosité de la composition cosmétique peut être ajustée pour maintenir au cours du temps les morceaux confits dispersés de façon homogène dans la composition.

Selon une variante, la viscosité de la composition peut être ajustée de façon à ce que - quand la composition est maintenue au repos - les morceaux confits reposent au fond du récipient (pot) dans lequel la composition est conditionnée, puis au moment de l'utilisation, après agitation pour remettre en suspension les morceaux confits dans la composition, la viscosité soit suffisante pour maintenir l'homogénéité de la composition le temps du prélèvement, et permettre ainsi à l'utilisateur de prélever la quantité suffisante de la composition pour un soin exfoliant.

Selon une mise en œuvre alternative, on peut imprégner les morceaux confits par une composition cosmétique.

Dans une autre mise en œuvre, l'invention a pour objet un kit dans lequel on conditionne séparément le ou les morceaux de fruit et la composition cosmétique appliquée sur la peau.

La présente demande divulgue un procédé de préparation d'une composition cosmétique comprenant :

- une étape de confisage d'un fruit, au cours de laquelle on réalise au moins une découpe du fruit, une mise en contact du fruit avec au moins un sirop de sucre, et au moins un égouttage des morceaux de fruits confits,
- une étape optionnelle de broyage des morceaux.confits de fruits obtenus à l'étape précédente,
- une étape de dispersion des morceaux confits dans une composition cosmétique.

Au cours de l'étape de confisage le fruit est plongé dans un sirop riche en sucre pour provoquer un échange entre l'eau du fruit et le sucre du sirop. Le fruit confit est ainsi simultanément enrichi en sucre et déshydraté. L'étape de confisage peut être réalisée plusieurs fois, en recyclant le sirop de sucre. Le sucre est de préférence le glucose, le fructose ou le sucre inverti (un mélange équimolaire des deux). Le fruit peut être frais, issu d'un stockage en saumure ou décongelé.

D'autres buts, caractéristiques et avantages de l'invention apparaîtront clairement à la lumière de la description explicative qui va suivre, faite en référence à des exemples de compositions cosmétiques utilisant ce nouvel agent exfoliant, donnés simplement à titre d'illustration.

Dans l'exemple ci-après, tous les pourcentages sont donnés en poids, la température est en degrés Celsius, la pression est la pression atmosphérique, sauf indication contraire.

**Exemple 1: Gel aqueux exfoliant contenant des morceaux d'écorce confite d'agrume**

Préparation des morceaux d'écorce confite d'agrume

On utilise un mélange d'écorce d'orange, de clémentine et de citron, en proportions égales.

Les morceaux d'écorces de taille grossière sont confits séparément en plusieurs étapes, par des sirops constitués d'eau, de fructose et de glucose dont la concentration en sucres augmente progressivement. Les sirops comprennent également un agent conservateur (anhydride sulfureux) et un correcteur d'acidité (acide citrique).

Après égouttage et refroidissement, les morceaux sont broyés pour obtenir des morceaux d'une taille d'au plus quelques millimètres.

Les morceaux d'écorces confites obtenus ont un degré Brix de 80 et un pH de 3.5.

Préparation du gel aqueux exfoliant contenant les morceaux d'agrume confit

On a préparé un gel aqueux de formule suivante. Les quantités sont exprimées en pourcentages massiques.

| | |
|---|---|
| Morceaux d'écorce confite d'Orange et de Clémentine | 25,0 |
| Morceaux d'écorce confite de Citron | 25,0 |
| Glycerol | 2,0 |
| Alginate de sodium | 1,5 |
| Phenoxyethanol | 0,3 |
| Tetrasodium EDTA | 0,2 |
| Tocopheryl acetate | 0,2 |
| Sodium citrate | 0,2 |
| Actifs | qs |
| Aqua | qsp 100,0 |

Le poids en écorces est de 50% en poids par rapport au poids de la composition, le solde étant constitué d'une formule de type gel aqueux translucide. La composition a l'aspect d'une marmelade dans laquelle les morceaux confits d'écorce sont dispersés de façon homogène.

Soin Exfoliant

On a réalisé des essais sur un panel de 11 sujets volontaires qui ont été sélectionnés parmi des femmes de plus de 18 ans ne présentant aucun désordre cutané.

Le matin du test, les sujets ont procédé à leur toilette avec leur produit nettoyant habituel, sans appliquer un produit de soin par la suite.

Pour évaluer l'effet exfoliant du produit de l'invention, on a utilisé des adhésifs commercialisés sous la référence D'Squame®. Le principe consiste à retirer les cellules mortes présentes à la surface de la peau, en collant un adhésif sur la peau, puis en le retirant. En décollant l'adhésif, celui-ci entraîne avec lui des morceaux des premières couches superficielles de la peau (encore appelées squames, lesquels sont constitués de cornéocytes). On peut ensuite analyser l'adhésif pour quantifier les squames qui ont été retirés de la peau.

Un adhésif n'est appliqué qu'une fois sur la peau. Il est sous la forme d'un disque fin de 22 mm de diamètre.

Pour chaque mesure on a collé un disque adhésif sur une zone de peau ; le disque a été maintenu pendant 10 secondes à l'aide d'un applicateur à 300 gf (2,942 N), puis décollé de la peau.

On a ensuite enregistré une image du disque à l'aide d'une caméra, d'un microscope optique et d'un éclairage standardisé. Un logiciel de traitement d'images permet de calculer un coefficient (CAI) qui quantifie le nombre et la distribution des squames présents sur le disque qui ont été retirés de la surface de la peau.

Le coefficient CAI est calculé par un logiciel sur la base de trois paramètres : le niveau de gris du disque (GL), le pourcentage des squames occupant la surface du disque (%Scales) et le pourcentage des cornéocytes occupant la surface du disque (%Corneo), selon la formule ci-dessous :

$$CAI = 1.5 \; x \; \frac{[GL - (100 - \%Scales)x2.0]x100}{\%Scales} + 100 \; x \; \frac{\%Corneo}{\%Scales}$$

Pour chaque sujet, on reproduit trois fois la mesure avec un nouveau disque pour une zone donnée et à un temps donné. La valeur retenue du CAI est la moyenne des trois mesures.

Sur une Zone 1, destinée à être traitée, on réalise une première mesure du CAI avant application du soin (CAIZ1 T0), puis une deuxième mesure du CAI après le soin (CAIZ1 T1). Le soin exfoliant a consisté à appliquer le gel aqueux décrit précédemment à l'aide d'un doigtier sur la zone de peau concerné (Zone 1), à laisser poser le gel 10 minutes sur la peau, puis à le rincer à l'eau.

Sur une zone de référence Zone 0 adjacente à la Zone 1, on a réalisé une première mesure du CAI (CAIZO T0) à T0, puis une deuxième mesure du CAI à T1 (CAIZO T1), sachant qu'aucun produit n'est appliqué sur cette zone.

Pour chaque sujet, on calcule donc :

- le CAI sur une Zone 1 traitée, avant application du soin sur la Zone 1 (CAIZ1 T0), et après application et rinçage du soin sur la Zone 1 (CAIZ1 T1)
- le CAI sur une Zone 0 non traitée adjacente à la Zone 1 de la peau, au temps T0 (CAIZO TO), et au temps T1 (CAIZO T1).

L'effet exfoliant du soin (noté VAR) a été évalué en mesurant la diminution du taux de squames prélevés après application du soin, c'est-à-dire la diminution du CAIZ1 T1 par rapport au CAIZ1 T0, après correction à l'aide de données collectées sur la zone Z0 non traitée, adjacente à la zone traitée Z1.

$$VAR \; \% = 100* \; [(CAIZ1 \; T1 - CAIZ1 \; T0) - (CAIZO \; T1 - CAIZO \; T0)] \; / \; CAIZO \; T1$$

Dans cette étude VAR est de 31% en moyenne pour l'ensemble du panel. L'étude statistique des résultats a confirmé que ce résultat était significatif.

En conclusion, la composition de l'invention a un effet exfoliant de la peau significatif.

## Exemple 2: Gel aqueux exfoliant contenant des morceaux d'écorce confite de citron

Préparation des morceaux d'écorce confite de citron

Les morceaux d'écorces de citron sont confits selon le même procédé que celui décrit à l'Exemple 1.

La teneur en furocoumarines des morceaux d'écorce confite de citron est déterminée, avant qu'ils soient utilisés pour la préparation d'un soin cosmétique exfoliant.

Dosage des furocoumarines par HPLC/UV/Fluorimétrie

Par HPLC:

Conditions chromatographiques:

| | |
|---|---|
| Colonne : | C18 Gravity Macherey Nagel, 250mm $\times$ 4 mm $\times$ 5 $\mu$m |
| Phase mobile : | A : Eau/ACN/THF (85/10/5 v/v) |
| | B : MeOH/ACN/THF (30/65/5 v/v) |
| Gradient : | 100% A ---> 100% A --->75% A ---> 75% A ---> 70% A ---> 15% A ---> 5% A |
| | 4 min      13 min      6 min      7 min      5 min      10 min |
| Débit : | 1 mL/min |
| Volume d'injection : | 5 $\mu$L |

(suite)

Détection : UV à 310 nm
Fluorimétrique : excitation à 310 nm, émission à 490 nm.

Préparation de la solution standard (0.08 µg/mL) :

Dans une fiole jaugée de 250 mL, on introduit 10 mg des trois furocoumarines suivantes :

- 8-methoxypsoralen    CAS 298-81-7
- psoralène    CAS 66-97-7
- bergaptène    CAS 484-20-8

La fiole est complétée au volume avec de l'acétonitrile. La solution est ensuite diluée à 0,2 mL dans 100 mL d'acétonitrile.

Préparation de la solution échantillon (80 mg/ml) :

Dans une fiole de 5 mL, introduire exactement 400 mg d'échantillon.
La fiole est complétée au volume avec de l'acétonitrile. La solution est ensuite dispersée sous agitation magnétique et aux ultra-sons.
Les solutions sont ensuite filtrées sur 0.45 µm, puis injectées dans l'appareil HPLC.

Résultats :

Les trois furocoumarines sont détectées à une teneur inférieure à 1 ppm.

Préparation du gel aqueux exfoliant contenant les morceaux d'écorce confite de citron

Les morceaux d'écorce confite de citron sont ensuite utilisés tels quels dans un gel aqueux exfoliant tel que décrit à l'Exemple 1.

**Revendications**

1. Utilisation de morceaux d'agrume obtenus par déshydratation osmotique ménagée dudit agrume dans un sirop de sucre, pour l'exfoliation mécanique des cellules mortes de l'épiderme de la peau d'une personne, lesdits morceaux d'agrume comprenant l'écorce et/ou l'épicarpe dudit agrume et ayant une teneur en composés furocoumarines inférieure à 50 ppm.

2. Utilisation selon la revendication 1, **caractérisée en ce que** lesdits morceaux d'agrume ont une taille comprise entre 0,1 mm et 5 cm.

3. Utilisation selon la revendication 1 ou la revendication 2, **caractérisée en ce que** lesdits morceaux d'agrume ont une teneur en composés furocoumarines inférieure à 20 ppm, préférentiellement inférieure à 10 ppm, plus préférentiellement inférieure à 5 ppm, et encore plus préférentiellement inférieure à 1 ppm.

4. Utilisation selon l'une des revendications 1 à 3, **caractérisée en ce que** lesdits morceaux d'agrume sont imprégnés d'une composition cosmétique comprenant un liquide choisi dans le groupe constitué de l'eau, d'une huile ou d'un mélange d'eau et d'huile, et au moins un excipient choisi parmi les surfactants et les gélifiants.

5. Utilisation selon l'une des revendications 1 à 4, **caractérisée en ce que** lesdits morceaux d'agrume se présentent sous la forme d'une composition cosmétique dans laquelle lesdits morceaux d'agrume sont répartis de manière homogène, ladite composition comprenant un liquide choisi dans le groupe constitué de l'eau, d'une huile ou d'un mélange d'eau et d'huile, et au moins un excipient choisi parmi les surfactants et les gélifiants.

6. Utilisation selon l'une des revendications 1 à 5, **caractérisée en ce que** l'agrume est choisi parmi les citrons, les clémentines, les kumquats, les bergamotes, les limes, les limettes, les mandarines, les oranges, les pamplemousses, les pomelos, les tangerines, les combavas, les yuzus, les cédrats et le calamondin.

**7.** Soin cosmétique exfoliant comprenant des morceaux d'agrume obtenus par déshydratation osmotique ménagée dudit agrume dans un sirop de sucre, à titre d'agent d'exfoliation mécanique, lesdits morceaux d'agrume comprenant l'écorce et/ou l'épicarpe dudit agrume et ayant une teneur en composés furocoumarines inférieure à 50 ppm, et ledit soin comprenant au moins un copolymère acrylique en tant que gélifiant hydrophile.

**8.** Soin cosmétique exfoliant selon la revendication 7, **caractérisé en ce que** lesdits morceaux d'agrume ont une taille comprise entre 0,1 mm et 5 cm.

**9.** Soin cosmétique exfoliant selon la revendication 7 ou la revendication 8, **caractérisé en ce qu'**il a une teneur en composés furocoumarines inférieure à 20 ppm, préférentiellement inférieure à 10 ppm, plus préférentiellement inférieure à 5 ppm, et encore plus préférentiellement inférieure à 1 ppm.

**10.** Soin cosmétique exfoliant selon l'une des revendications 7 à 9, **caractérisé en ce que** les morceaux d'agrume sont imprégnés d'une composition cosmétique comprenant un liquide choisi dans le groupe constitué de l'eau, d'une huile ou d'un mélange d'eau et d'huile, et au moins un excipient choisi parmi les surfactants et les gélifiants.

**11.** Soin cosmétique exfoliant selon l'une des revendications 7 à 10, **caractérisé en ce qu'**il se présente sous la forme d'une composition cosmétique dans laquelle sont répartis les morceaux d'agrume de manière homogène, ladite composition comprenant un liquide choisi dans le groupe constitué de l'eau, d'une huile ou d'un mélange d'eau et d'huile, et au moins un excipient choisi parmi les surfactants et les gélifiants.

**12.** Soin cosmétique exfoliant selon l'une des revendications 7 à 11, **caractérisé en ce que** l'agrume est choisi parmi les citrons, les clémentines, les kumquats, les bergamotes, les limes, les limettes, les mandarines, les oranges, les pamplemousses, les pomelos, les tangerines, les combavas, les yuzus, les cédrats et le calamondin.

**13.** Kit cosmétique exfoliant comprenant

a) une composition cosmétique comprenant un liquide choisi dans le groupe constitué de l'eau, une huile ou d'un mélange d'eau et d'huile, et au moins un copolymère acrylique en tant que gélifiant hydrophile, et
b) des morceaux d'agrume obtenus par déshydratation osmotique ménagée dudit agrume dans un sirop de sucre, lesdits morceaux d'agrume comprenant l'écorce et/ou l'épicarpe dudit agrume et ayant une teneur en composés furocoumarines inférieure à 50 ppm,

la composition et les morceaux d'agrume étant conditionnés de façon séparée dans un même emballage.

**14.** Kit cosmétique exfoliant comprenant selon la revendication 13, **caractérisé en ce que** lesdits morceaux d'agrume ont une taille comprise entre 0,1 mm et 5 cm.

**15.** Procédé d'exfoliation de la peau d'une personne comprenant les étapes consistant à :

- appliquer, sur la peau d'une personne, des morceaux d'agrume obtenus par déshydratation osmotique ménagée dudit agrume dans un sirop de sucre, lesdits morceaux d'agrume comprenant l'écorce et/ou l'épicarpe dudit agrume et ayant une teneur en composés furocoumarines inférieure à 50 ppm,
- masser la peau recouverte desdits morceaux par frottement de la surface de la peau, de manière à exfolier mécaniquement les cellules mortes qui s'y trouvent,
- rincer ou essuyer la peau pour ôter les morceaux confits d'agrume et les cellules mortes exfoliées.

**16.** Procédé d'exfoliation de la peau selon la revendication 15, **caractérisé en ce que** lesdits morceaux d'agrume ont une taille comprise entre 0,1 mm et 5 cm.

**Patentansprüche**

**1.** Verwendung von Stücken einer Zitrusfrucht, die durch schonende osmotische Trocknung der Zitrusfrucht in einem Zuckersirup erhalten werden, zur mechanischen Abschälung von abgestorbenen Epidermiszellen der Haut einer Person, wobei die Stücke der Zitrusfrucht die Schale und/oder das Exokarp der Zitrusfrucht umfassen und einen Gehalt an Furocumarinverbindungen von unter 50 ppm aufweisen.

2. Verwendung nach Anspruch 1, **dadurch gekennzeichnet, dass** die Stücke der Zitrusfrucht eine Größe von zwischen 0,1 mm und 5 cm aufweisen.

3. Verwendung nach Anspruch 1 oder Anspruch 2, **dadurch gekennzeichnet, dass** die Stücke der Zitrusfrucht einen Gehalt an Furocumarinverbindungen von unter 20 ppm, bevorzugt unter 10 ppm, noch bevorzugter unter 5 ppm und sogar noch bevorzugter unter 1 ppm aufweisen.

4. Verwendung nach einem der Ansprüche 1 bis 3, **dadurch gekennzeichnet, dass** die Stücke der Zitrusfrucht mit einer kosmetischen Zusammensetzung getränkt sind, die eine Flüssigkeit, die ausgewählt ist aus der Gruppe bestehend aus Wasser, einem Öl oder einem Gemisch aus Wasser und Öl, und mindestens einen Hilfsstoff, der aus Tensiden und Geliermitteln ausgewählt ist, umfasst.

5. Verwendung nach einem der Ansprüche 1 bis 4, **dadurch gekennzeichnet, dass** die Stücke der Zitrusfrucht in Form einer kosmetischen Zusammensetzung vorliegen, in welcher die Stücke der Zitrusfrucht auf homogene Weise verteilt sind, wobei die Zusammensetzung eine Flüssigkeit, die ausgewählt ist aus der Gruppe bestehend aus Wasser, einem Öl oder einem Gemisch aus Wasser und Öl, und mindestens einen Hilfsstoff, der aus Tensiden und Geliermitteln ausgewählt ist, umfasst.

6. Verwendung nach einem der Ansprüche 1 bis 5, **dadurch gekennzeichnet, dass** die Zitrusfrucht ausgewählt ist aus Zitronen, Clementinen, Kumquats, Bergamotten, Limonen, Limetten, Mandarinen, Orangen, Pampelmusen, Pomelos, Tangerinen, Kaffernlimetten, Yuzus, Zitronatzitronen und Calamondinorangen.

7. Kosmetische Schälkur, umfassend Stücke einer Zitrusfrucht, die durch schonende osmotische Trocknung der Zitrusfrucht in einem Zuckersirup erhalten werden, als mechanisches Schälmittel, wobei die Stücke der Zitrusfrucht die Schale und/oder das Exokarp der Zitrusfrucht umfassen und einen Gehalt an Furocumarinverbindungen von unter 50 ppm aufweisen und wobei die Kur mindestens ein Acrylcopolymer als hydrophiles Geliermittel umfasst.

8. Kosmetische Schälkur nach Anspruch 7, **dadurch gekennzeichnet, dass** die Stücke der Zitrusfrucht eine Größe von zwischen 0,1 mm und 5 cm aufweisen.

9. Kosmetische Schälkur nach Anspruch 7 oder Anspruch 8, **dadurch gekennzeichnet, dass** sie einen Gehalt an Furocumarinverbindungen von unter 20 ppm, bevorzugt unter 10 ppm, noch bevorzugter unter 5 ppm und sogar noch bevorzugter unter 1 ppm aufweist.

10. Kosmetische Schälkur nach einem der Ansprüche 7 bis 9, **dadurch gekennzeichnet, dass** die Stücke der Zitrusfrucht mit einer kosmetischen Zusammensetzung getränkt sind, die eine Flüssigkeit, die ausgewählt ist aus der Gruppe bestehend aus Wasser, einem Öl oder einem Gemisch aus Wasser und Öl, und mindestens einen Hilfsstoff, der aus Tensiden und Geliermitteln ausgewählt ist, umfasst.

11. Kosmetische Schälkur nach einem der Ansprüche 7 bis 10, **dadurch gekennzeichnet, dass** sie in Form einer kosmetischen Zusammensetzung vorliegt, in welcher die Stücke der Zitrusfrucht auf homogene Weise verteilt sind, wobei die Zusammensetzung eine Flüssigkeit, die ausgewählt ist aus der Gruppe bestehend aus Wasser, einem Öl oder einem Gemisch aus Wasser und Öl, und mindestens einen Hilfsstoff, der aus Tensiden und Geliermitteln ausgewählt ist, umfasst.

12. Kosmetische Schälkur nach einem der Ansprüche 7 bis 11, **dadurch gekennzeichnet, dass** die Zitrusfrucht ausgewählt ist aus Zitronen, Clementinen, Kumquats, Bergamotten, Limonen, Limetten, Mandarinen, Orangen, Pampelmusen, Pomelos, Tangerinen, Kaffernlimetten, Yuzus, Zitronatzitronen und Calamondinorangen.

13. Kosmetisches Schälkur-Kit, umfassend

a) eine kosmetische Zusammensetzung, umfassend eine Flüssigkeit, die ausgewählt ist aus der Gruppe, bestehend aus Wasser, einem Öl oder einem Gemisch aus Wasser und Öl, und mindestens ein Acrylcopolymer als hydrophiles Geliermittel, und
b) Stücke einer Zitrusfrucht, die durch schonende osmotische Trocknung der Zitrusfrucht in einem Zuckersirup erhalten werden, wobei die Stücke der Zitrusfrucht die Schale und/oder das Exokarp der Zitrusfrucht umfassen und einen Gehalt an Furocumarinverbindungen von unter 50 ppm aufweisen,

wobei die Zusammensetzung und die Stücke der Zitrusfrucht getrennt in einer selben Verpackung abgepackt sind.

14. Kosmetisches Schälkur-Kit nach Anspruch 13, **dadurch gekennzeichnet, dass** die Stücke der Zitrusfrucht eine Größe von zwischen 0,1 mm und 5 cm aufweisen.

15. Verfahren zum Abschälen der Haut einer Person, umfassend die Schritte, bestehend aus:

- Anwenden, auf die Haut einer Person, von Stücken einer Zitrusfrucht, die durch schonende osmotische Trocknung der Zitrusfrucht in einem Zuckersirup erhalten werden, wobei die Stücke der Zitrusfrucht die Schale und/oder das Exokarp der Zitrusfrucht umfassen und einen Gehalt an Furocumarinverbindungen von unter 50 ppm aufweisen,
- Massieren der mit den Stücken bedeckten Haut durch Reiben der Oberfläche der Haut derart, dass dort befindliche abgestorbene Zellen mechanisch abgeschält werden,
- Spülen oder Abwischen der Haut, um die kandierten Stücke der Zitrusfrucht und die abgeschälten abgestorbenen Zellen zu entfernen.

16. Verfahren zum Abschälen der Haut nach Anspruch 15, **dadurch gekennzeichnet, dass** die Stücke der Zitrusfrucht eine Größe von zwischen 0,1 mm und 5 cm aufweisen.

**Claims**

1. The use of citrus fruit pieces obtained by controlled osmotic dehydration of said citrus fruit in a sugar syrup, for mechanically exfoliating dead cells from the epidermis of the skin of an individual, said citrus fruit pieces comprising the peel and/or the epicarp of said citrus fruit and having a furocoumarin compound content of less than 50 ppm.

2. The use as claimed in claim 1, **characterized in that** said citrus fruit pieces have a size of between 0.1 mm and 5 cm.

3. The use as claimed in claim 1 or claim 2, **characterized in that** said citrus fruit pieces have a furocoumarin compound content of less than 20 ppm, preferentially less than 10 ppm, more preferentially less than 5 ppm, and even more preferentially less than 1 ppm.

4. The use as claimed in one of claims 1 to 3, **characterized in that** said citrus fruit pieces are impregnated with a cosmetic composition comprising a liquid chosen from the group consisting of water, an oil or a mixture of water and oil, and at least one excipient chosen from surfactants and gelling agents.

5. The use as claimed in one of claims 1 to 4, **characterized in that** said citrus fruit pieces are in the form of a cosmetic composition in which said citrus fruit pieces are uniformly distributed, said composition comprising a liquid chosen from the group consisting of water, an oil or a mixture of water and oil, and at least one excipient chosen from surfactants and gelling agents.

6. The use as claimed in one of claims 1 to 5, **characterized in that** the citrus fruit is chosen from lemons, clementines, kumquats, bergamots, limes, Tahiti limes, mandarins, oranges, grapefruits, pomelos, tangerines, kaffir limes, yuzus, citrons and calamondin.

7. An exfoliating cosmetic care product comprising citrus fruit pieces obtained by controlled osmotic dehydration of said citrus fruit in a sugar syrup, as a mechanical exfoliating agent, said citrus fruit pieces comprising the peel and/or the epicarp of said citrus fruit and having a furocoumarin compound content of less than 50 ppm, and said care product comprising at least one acrylic copolymer as hydrophilic gelling agent.

8. The exfoliating cosmetic care product as claimed in claim 7, **characterized in that** said citrus fruit pieces have a size of between 0.1 mm and 5 cm.

9. The exfoliating cosmetic care product as claimed in claim 7 or claim 8, **characterized in that** it has a furocoumarin compound content of less than 20 ppm, preferentially less than 10 ppm, more preferentially less than 5 ppm, and even more preferentially less than 1 ppm.

10. The exfoliating cosmetic care product as claimed in one of claims 7 to 9, **characterized in that** the citrus fruit pieces are impregnated with a cosmetic composition comprising a liquid chosen from the group consisting of water, an oil or a mixture of water and oil, and at least one excipient chosen from surfactants and gelling agents.

11. The exfoliating cosmetic care product as claimed in one of claims 7 to 10, **characterized in that** it is in the form of a cosmetic composition in which the citrus fruit pieces are uniformly distributed, said composition comprising a liquid chosen from the group consisting of water, an oil or a mixture of water and oil, and at least one excipient chosen from surfactants and gelling agents.

12. The exfoliating cosmetic care product as claimed in one of claims 7 to 11, **characterized in that** the citrus fruit is chosen from lemons, clementines, kumquats, bergamots, limes, Tahiti limes, mandarins, oranges, grapefruits, pomelos, tangerines, kaffir limes, yuzus, citrons and calamondin.

13. An exfoliating cosmetic kit comprising

a) a cosmetic composition comprising a liquid chosen from the group consisting of water, an oil or a mixture of water and oil, and at least one acrylic copolymer as hydrophilic gelling agent, and
b) citrus fruit pieces obtained by controlled osmotic dehydration of said citrus fruit in a sugar syrup, said citrus fruit pieces comprising the peel and/or the epicarp of said citrus fruit and having a furocoumarin compound content of less than 50 ppm,

the composition and the citrus fruit pieces being packaged separately in one and the same packaging.

14. The exfoliating cosmetic kit comprising as claimed in claim 13, **characterized in that** said citrus fruit pieces have a size of between 0.1 mm and 5 cm.

15. A method for exfoliating the skin of an individual, comprising the steps consisting in:

- applying, to the skin of an individual, citrus fruit pieces obtained by controlled osmotic dehydration of said citrus fruit in a sugar syrup, said citrus fruit pieces comprising the peel and/or the epicarp of said citrus fruit and having a furocoumarin compound content of less than 50 ppm,
- massaging the skin covered with said pieces by rubbing the surface of the skin, so as to mechanically exfoliate the dead cells located thereon,
- rinsing or wiping the skin in order to remove the candied citrus fruit pieces and the exfoliated dead cells.

16. The method for exfoliating the skin as claimed in claim 15, **characterized in that** said citrus fruit pieces have a size of between 0.1 mm and 5 cm.

**EP 3 331 617 B1**

## RÉFÉRENCES CITÉES DANS LA DESCRIPTION

**Documents brevets cités dans la description**

- US 20040091446 A1